# EUROPÄISCHE PATENTSCHRIFT

(11) **EP 3 911 257 B1**
(45) Veröffentlichungstag und Bekanntmachung des Hinweises auf die Patenterteilung: **11.03.2026**
(21) Anmeldenummer: 19818061.4
(22) Anmeldetag: 10.12.2019
(51) Int. Cl.: A61B 17/86, A61F 2/08

(54) **KNOCHENTRANSPLANTAT**
BONE TRANSPLANT
GREFFON OSSEUX

(30) Priorität: 16.01.2019 AT 500342019
(43) Veröffentlichungstag der Anmeldung: 24.11.2021
(73) Patentinhaber: Surgebright GmbH, 4040 Lichtenberg (AT)
(72) Erfinder: PASTL, Klaus, 4040 Lichtenberg (AT)
(74) Vertreter: KLIMENT & HENHAPEL
(86) Internationale Anmeldenummer: PCT/EP2019/084519
(87) Internationale Veröffentlichungsnummer: WO 2020/148025

(56) Entgegenhaltungen:
- EP-A1- 2 384 712
- EP-B1- 2 384 712
- US-A1- 2017 231 674
- US-A1- 2018 028 236

## Beschreibung

Die Erfindung betrifft einen Sehnenanker zur Refixation von Sehnen mit einem zylindrischen oder kegelstumpfförmigen Schraubenschaft und einem Schraubenkopf zur Einleitung eines Eindrehmoments, wobei sowohl der Schraubenschaft als auch der Schraubenkopf mit einem Außengewinde versehen sind, gemäß Anspruch 1.

Schrauben für chirurgische Verfahren werden in herkömmlicher Weise aus Metall bzw. Metalllegierungen gefertigt. Ferner sind Schrauben aus resorbierbarem Material, etwa Polyglykolid und Polylaktid, bekannt. Schrauben dieser Art weisen in der chirurgischen Praxis allerdings mehrere Nachteile auf. So müssen etwa Schrauben aus Metall bzw. Metalllegierungen einerseits durch eine Zweitoperation wieder entfernt werden, und unterliegen andererseits Veränderungen durch Korrosion. Damit erhöhen sich die Kosten im Gesundheitssystem, sowie die gesundheitlichen Risiken für jeden Patienten durch eine neuerliche Operation. Sämtliche resorbierbaren Materialien werden zwar aufgelöst, können aber während ihres Abbaus zu großen Osteolysen in dem umgebenden Knochen führen, also zu einem Wegweichen des Empfängerknochens von der Schraube. Osteolytische Prozesse können zu einer Lockerung und schließlich zu einem Ausbrechen des Implantats führen. Die Bioresorption wiederum ist ein jahrelanger Prozess, der von Entzündungen und Schmerzen begleitet sein kann. In der Druckschrift US 2017/0231674 A1 wird eine Schraube für die Fixierung von Knochenbrüchen beschrieben. In der US 2018/0028236 A1 wird eine Vorrichtung zur Stabilisierung von Wirbelknochen beschrieben.

Schrauben aus allogenem Knochen (Femur und Tibia-Corticalis), wie sie etwa aus der PCT/EP2018/071619 der Anmelderin bekannt sind, die für die vorliegende Erfindung als nächstkommender Stand der Technik betrachtet wird, verfügen hingegen über mehrere Vorteile. Sie werden ohne Abstoßungsreaktion vaskularisiert und umgebaut, und sind beispielsweise für Osteosynthesen geeignet, wo etwa kleine Knochenfragmente zusammengefügt werden müssen, da durch die Schraube bereits bei der Operation eine tragende Knochenbrücke entsteht, die sich vom Zeitpunkt der Operation an verbessert, indem sie sich umbaut und voll in den lebenden Knochen integriert und eingebaut wird. Schrauben mit einem Durchmesser von 3-4 mm etwa werden innerhalb von zwei Monaten vollständig mit Gefäßen durchwachsen. Diese Knochenschrauben können daher auch als Knochentransplantate bezeichnet werden. Im Gegensatz dazu stellen Metallschrauben eher ein Hindernis für die Knochenneubildung dar, insbesondere verringern sie durch deren bloße Präsenz die zur Verfügung stehende Oberfläche, die für die Knochenheilung vorhanden wäre. Abbaubare Materialien wiederum haben ihre maximale Festigkeit zum Zeitpunkt der Operation. Für sie gelten dieselben Nachteile wie für die Metallschrauben, des Weiteren nimmt die Festigkeit rapide ab, sobald der Abbauprozess eintritt, wodurch die zu osteosynthetisierende Knochenstelle zumindest vorübergehend wieder eine Schwächung erfährt.

Diese Nachteile zeigen sich insbesondere bei der Herstellung von Sehnenanker. Sehnenanker werden zur Refixation von Sehnen an Knochen verwendet. Hierbei wird in eine vorgefertigte Bohrung eine in herkömmlicher Weise aus Metall gefertigte Schraube mithilfe eines Eindrehwerkzeuges eingedreht, wobei das Sehnenende durch Reibschluss zwischen der vorgefertigten Bohrung und der eingesetzten Schraube befestigt wird. Die Verwendung eines Sehnenankers aus einem metallischen Material oder aus einem Kunststoff verringert die für ein Anwachsen der Sehne zur Verfügung stehende Oberfläche und verursacht störende Artefakte in nachfolgenden bildgebenden Verfahren wie MRT und CT.

Bei Verwendung von Knochentransplantaten aus allogenem Knochen könnten diese Nachteile vermieden werden, da auch die Oberfläche des Knochentransplantats ein Anwachsen der Sehne erlaubt und die für das Anwachsen der Sehne zur Verfügung stehende Oberfläche somit deutlich erhöht. Allerdings muss bei der Anwendung von Schrauben aus allogenem Knochen in der chirurgischen Praxis beachtet werden, dass sie sich hinsichtlich Eindrehwiderstand und Festigkeit erheblich von Metallschrauben unterscheiden. Da sie aus allogener, humaner Corticalis gewonnen werden, ist nicht zu erwarten, dass Kenntnisse zu Gewindeformen, Eindrehwiderstand, Rotationsstabilität oder Festigkeit wie sie von Schrauben aus Metall bekannt sind, ohne weiteres übertragbar sind.

Eine weitere Herausforderung bei der Verwendung von Knochentransplantaten als Sehnenanker besteht des Weiteren in den Belastungen, die von der Sehne auf das Knochentransplantat ausgeübt werden. Das Knochentransplantat muss trotz der Belastungen einen sicheren Halt der Sehne gewährleisten und eine hohe Rotationsstabilität aufweisen, also eine hohe Selbsthemmung gegenüber einer ungewollten Rotation und somit einem Lösen der Schraube. Durch Verwendung einer möglichst geringen Gewindesteigung, und somit möglichst vieler Windungen desselben Gewindeganges pro axialer Längeneinheit, könnte die Oberfläche für ein Anwachsen der Sehne vergrößert werden, was zu einem besseren Halt der Sehne führt. Zudem kann auf diese Weise die Rotationsstabilität erhöht werden. Andererseits wird das Sehnengewebe beim Einschrauben des Knochentransplantats umso stärker belastet, je kleiner die Gewindesteigung ist, da die Bindegewebsfasern der Sehne in diesem Fall dazu neigen aufgetrennt zu werden und die Sehne in ihren Befestigungspunkten am Knochen an Festigkeit verliert. Es wäre stattdessen vorteilhaft, die zwischen Sehnenanker und dem umgebenden Knochen befindlichen Sehnenteile während des Einschraubens so gering wie möglich zu belasten, um Schädigungen des Sehnengewebes zu vermeiden. Zudem wird die für das Einsetzen der Schraube erforderliche Anzahl an Umdrehungen umso höher, je niedriger die Gewindesteigung der Schraube ist. Das erweist sich in der chirurgischen Praxis mitunter als problematisch, insbesondere bei schwer zugänglichen Stellen. Dieser Sachverhalt wird im Folgenden auch als Eindrehverhalten bezeichnet, wobei sich ein gutes Eindrehverhalten durch ein rasches und leichtes Eindrehen der Schraube auszeichnet.

Es ist somit das Ziel der Erfindung Knochentransplantate aus kortikalem Knochen für einen Einsatz als Sehnenanker zu optimieren. Insbesondere sollen sie eine gute Fixierung der Sehne gewährleisten und rasch implantierbar sein.

Diese Ziele werden durch die Merkmale von Anspruch 1 erreicht. Anspruch 1 bezieht sich auf einen Sehnenanker zur Refixation von Sehnen mit einem zylindrischen oder kegelstumpfförmigen Schraubenschaft und einem Schraubenkopf zur Einleitung eines Eindrehmoments, wobei sowohl der Schraubenschaft als auch der Schraubenkopf mit einem Außengewinde versehen sind. Erfindungsgemäß wird hierbei vorgeschlagen, dass der Sehnenanker als bohrungsfreies Knochentransplantat aus einem kortikalen Knochenmaterial gefertigt ist, wobei es sich zumindest beim Außengewinde des Schraubenschaftes um ein mehrgängiges Gewinde handelt und axiale Ausnehmungen in den Außenmantel des Schraubenkopfes eingefräst sind, die in die proximale Stirnfläche des Schraubenkopfes münden, wobei die Länge des Knochentransplantats zumindest dreimal so groß wie der Durchmesser des Knochentransplantats im Fall einer zylindrischen Ausführung ist, und zumindest dreimal so groß wie der größte Durchmesser des Knochentransplantats im Fall einer kegelstumpfförmigen Ausführung des Knochentransplantats.

Bei mehrgängigen Gewinden sind zumindest zwei Gewindegänge parallel um den Schraubenschaft "gewickelt". Auf diese Weise kann die Steigung eines jeden Gewindeganges und somit der Gewindehub der Schraube vergrößert werden. Der von einem Gewindegang nicht benötigte Zwischenraum wird von einem zweiten Gewindegang oder weiteren Gewindegängen gefüllt. Auf diese Weise kann einerseits ein bestimmter Hub mit weniger Umdrehungen beziehungsweise in kürzerer Zeit erreichbar sein, wodurch das Eindrehverhalten verbessert und das Sehnengewebe geschont wird. Andererseits steht durch die Verwendung mehrerer Gewindegänge eine hohe Oberfläche für das Anwachsen des Sehnengewebes am Sehnenanker zur Verfügung, wodurch die Fixierung der Sehne verbessert und die Rotationsstabilität erhöht wird. Das erfindungsgemäße Knochentransplantat gewährleistet somit eine gute Fixierung der Sehne und ist rasch implantierbar.

Gemäß einer möglichen Ausführungsform wird etwa vorgeschlagen, dass das mehrgängige Gewinde zwei Gewindegänge umfasst, die jeweils eine Steigung zwischen 0.8 mm und 3 mm aufweisen. Das erfindungsgemäße Knochentransplantat ist hierfür in seinen Abmessungen zäpfchenförmig ausgeführt und weist somit proximal einen zylindrischen Abschnitt auf, der distal in einen konischen Abschnitt übergeht. Das Verhältnis von Gewindetiefe t zu Gewindeaußendurchmesser D liegt vorzugsweise zwischen 0.10 und 0.25.

Vorzugsweise handelt es sich bei dem Gewinde um ein Spitzgewinde. Das Spitzgewinde optimiert nicht nur die für das Anwachsen des Sehnengewebes am Sehnenanker zur Verfügung stehende Oberfläche, sondern bietet auch praktische Vorteile in der Herstellung des erfindungsgemäßen Knochentransplantats, da das Spitzgewinde mit geringerem Platzbedarf entlang des Schraubenumfanges angeordnet werden kann.

Erfindungsgemäß ist auch der Schraubenkopf mit einem Außengewinde versehen und trägt somit zur Festigkeit der Sehnenbefestigung bei. Insbesondere kann auch der Schraubenkopf in den Knochen eingedreht werden ohne abgeschnitten werden zu müssen. Zur Einleitung eines Eindrehmoments wird vorzugsweise vorgeschlagen, dass zumindest zwei um die Schraubenkopfachse verteilt angeordnete, in Richtung der Schraubenkopfachse axial verlaufende und in die Stirnfläche des freien Endes des Schraubenkopfes mündende Ausnehmungen zur Aufnahme eines Eindrehwerkzeuges vorgesehen sind, wobei die Ausnehmungen jeweils durch Seitenflächen gebildet werden, die sich von einer das Außengewinde des Schraubenkopfes einhüllenden Außenmantelfläche in Richtung der Schraubenkopfachse erstrecken und in einem achsnahen Flächenabschnitt ineinander übergehen. Das Außengewinde des Schraubenkopfes wird somit lediglich von den zur Einleitung eines Eindrehmoments vorgesehenen, axial verlaufenden Ausnehmungen unterbrochen. Das Knochentransplantat ist ansonsten bohrungsfrei und besteht zur Gänze aus Knochenmaterial. Insbesondere im Bereich der Schraubenkopfachse verbleibt somit Knochenmaterial, es werden lediglich axiale Ausnehmungen in den Außenmantel des Schraubenkopfes eingefräst, die in die proximale Stirnfäche des Schraubenkopfes münden. In diese Ausnehmungen können axiale Fortsätze eines Eindrehwerkzeuges stirnseitig axial eingeschoben werden. Das Eindrehmoment wird in weiterer Folge auf die Seitenflächen der Ausnehmungen ausgeübt, wobei das Eindrehmoment auf kinematisch günstige Weise im äußeren Umfangsbereich des Schraubenkopfes eingebracht wird.

Weiters wird zur Erhöhung des Eindrehmoments vorgeschlagen, dass vier symmetrisch um die Schraubenkopfachse verteilt angeordnete Ausnehmungen vorgesehen sind. Im Zuge des axialen Einführens der Fortsätze des Eindrehwerkzeugs werden im Fall von vier symmetrisch um die Schraubenkopfachse verteilt angeordneten Ausnehmungen bei den für Knochenschrauben üblichen Durchmessern im einstelligen Millimeterbereich die zwischen den Ausnehmungen verbleibenden Schraubenkopfbereiche von den Fortsätzen des Eindrehwerkzeuges umgriffen und gewissermaßen "eingespannt", wodurch ein Abbrechen von Knochenmaterial unterbunden wird. Die vorgeschlagenen Ausnehmungen gestatten dabei auch ein Fluchten des äußeren Schraubenkopfdurchmessers mit dem äußeren Durchmesser des Schraubenschaftes sowie mit dem äußeren Durchmesser des Eindrehwerkzeuges und ermöglichen dadurch auch neue chirurgische Einsatzgebiete wie beispielsweise einen endoskopischen oder arthroskopischen Einsatz der Schraube.

Das Knochentransplantat kann zylindrisch oder kegelstumpfförmig ausgeführt sein. Der Gewindeaußendurchmesser des Knochentransplantats liegt dabei vorzugsweise zwischen 7,0mm und 4,5mm. Die Länge des Knochentransplantats ist dabei erfindungsgemäß zumindest dreimal so groß wie der Durchmesser des Knochentransplantats im Fall einer zylindrischen Ausführung und zumindest dreimal so groß wie der größte Durchmesser des Knochentransplantats im Fall einer kegelstumpfförmigen Ausführung des Knochentransplantats.

Die Erfindung wird Im Folgenden anhand von Ausführungsbeispielen mithilfe der beiliegenden Figuren näher erläutert. Hierbei zeigen die
Fig. 1 einen Gewindeabschnitt eines Spitzgewindes zur Erläuterung der maßgeblichen Gewindeparameter, und die
Fig. 2 eine Ausführungsform eines erfindungsgemäßen Knochentransplantats, wobei die
Fig. 2a das erfindungsgemäße Knochentransplantat von oben gesehen zeigt, sodass der Schraubenkopf ersichtlich ist,
Fig. 2b und 2c das erfindungsgemäße Knochentransplantat von der Seite gesehen zeigen, und die
Fig. 2d eine perspektivische Ansicht eines erfindungsgemäßen Knochentransplantats zeigt.

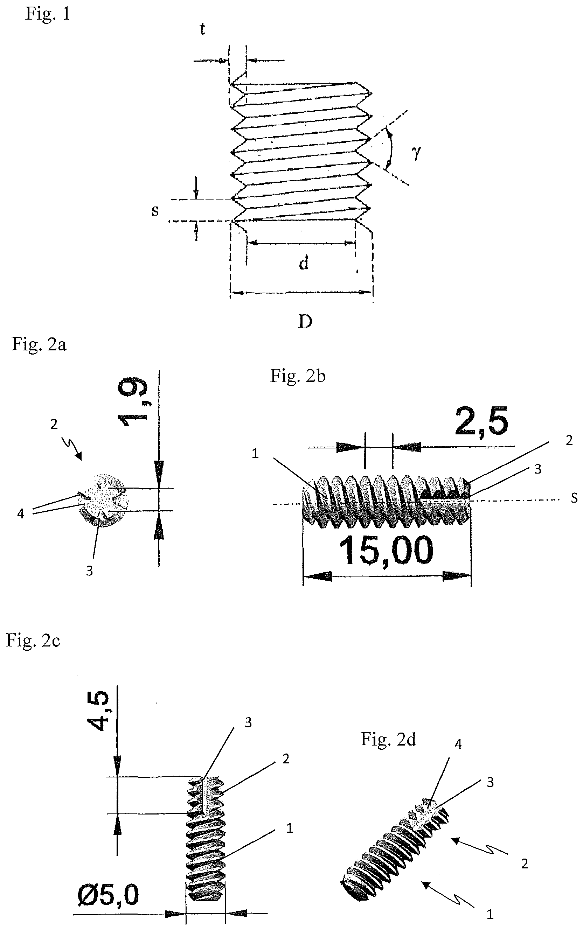

Anhand der Fig. 1 werden zunächst maßgebliche Gewindeparameter erläutert. Es handelt sich hierbei um den Gewindeaußendurchmesser D und den Kerndurchmesser d, wobei durch deren Differenz die Gewindetiefe t festgelegt ist, sowie die Steigung s des Gewindes, wobei die Steigung s jener Weg ist, der durch eine Umdrehung der Schraube zurück gelegt wird, also der Abstand zwischen zwei Gewindespitzen desselben Gewindeganges, der bei metrischen Gewinden in Millimeter angegeben wird. Der Kehrwert der Steigung s entspricht in der Regel der Anzahl der Windungen eines Gewindeganges pro Längeneinheit, also bei metrischen Gewinden der Anzahl der Windungen pro Millimeter. Ein weiterer Parameter ist der Flankenwinkel γ, der sich wiederum aus der Gewindetiefe t und der Steigung s ergibt.

Je größer der Flankenwinkel γ bei gleich bleibender Gewindetiefe t ist, desto weniger Windungen pro mm stehen für die Sehnenbefestigung zur Verfügung. Dadurch nehmen die Festigkeit der Sehnenbefestigung und die Rotationsstabilität der Schraube ab. Andererseits wird der Gewindehub vergrößert, sodass das Eindrehverhalten für die chirurgische Praxis begünstigt wird. Andererseits wird das Sehnengewebe beim Einschrauben des Knochentransplantats umso stärker belastet, je kleiner die Gewindesteigung ist. Mithilfe der erfindungsgemäßen Merkmale können diese widersprüchlichen Voraussetzungen zur Optimierung des Sehnenankers aufgelöst werden.

Hierzu wird auf die Fig. 2 eingegangen, die eine Ausführungsform eines Knochentransplantats aus einem kortikalen Knochenmaterial für die chirurgische Verwendung als Sehnenanker zeigt. Das Knochentransplantat weist einen zylindrischen Schraubenschaft 1 auf, der mit einem Außengewinde versehen ist, sowie einen Schraubenkopf 2 zur Einleitung eines Eindrehmoments, der auch einheilt und nicht weggeschnitten werden muss wie herkömmliche Schraubenköpfe. Der Schraubenkopf 2 ist ebenfalls mit einem Außengewinde versehen und weist eine das Außengewinde des Schraubenkopfes einhüllende Außenmantelfläche auf, die um die Schraubenkopfachse S rotationssymmetrisch ist. Der Schraubenkopf 2 weist ferner vier um die Schraubenkopfachse S verteilt angeordnete, in Richtung der Schraubenkopfachse S axial verlaufende und in die Stirnfläche des freien Endes des Schraubenkopfes 2 mündende Ausnehmungen 3 zur Aufnahme eines Eindrehwerkzeuges. Die axial verlaufenden Ausnehmungen 3 werden jeweils durch sich von der Außenmantelfläche in Richtung der Schraubenkopfachse S erstreckende Seitenflächen 4 gebildet, die in einem achsnahen Flächenabschnitt ineinander übergehen (siehe insbesondere Fig. 2a). Die von der Außenmantelfläche in Richtung der Schraubenkopfachse S verlaufenden Seitenflächen 4 der Ausnehmungen 3 können konvex ausgeführt sein, und der achsnahe Flächenabschnitt konkav.

Wie den Fig. 2a-2d zu entnehmen ist, erstreckt sich das Außengewinde bei der gezeigten Ausführungsform mit unveränderten Gewindeparametern sowohl über den Schraubenschaft 1, als auch über den Schraubenkopf 2. Der Schraubenkopf 2 trägt somit zur Festigkeit der Sehnenbefestigung bei. Insbesondere kann auch der Schraubenkopf 2 in den Knochen eingedreht werden ohne abgeschnitten werden zu müssen. Das Außengewinde wird lediglich von den zur Einleitung eines Eindrehmoments vorgesehenen, axial verlaufenden Ausnehmungen 3 unterbrochen. Im Bereich der Schraubenkopfachse S verbleibt somit Knochenmaterial mit einem Kerndurchmesser d, wie in der Fig. 2a ersichtlich ist, da lediglich axiale Ausnehmungen 3 in den Außenmantel des Schraubenkopfes 2 eingefräst werden, die in die proximale Stirnfäche des Schraubenkopfes 2 münden. In diese Ausnehmungen 3 können axiale Fortsätze eines Eindrehwerkzeuges stirnseitig axial eingeschoben werden. Das Eindrehmoment wird in weiterer Folge auf die Seitenflächen 4 der Ausnehmungen 3 ausgeübt.

Das Außengewinde ist erfindungsgemäß als mehrgängiges Gewinde ausgeführt. Gemäß der Ausführungsform der Fig. 2 ist das Außengewinde des Schraubenschaftes 1 und des Schraubenkopfes 2 etwa als ein mehrgängiges Gewinde mit zwei Gewindegängen ausgeführt, die jeweils eine Steigung von 2.5 mm aufweisen. Die Gesamtlänge der gezeigten Schraube beträgt 15 mm bei einem Gewindeaußendurchmesser D von 5 mm. Die axiale Länge der Ausnehmungen 3 des Schraubenkopfes 2 beträgt 4.5 mm, wobei der verbleibende Kerndurchmesser d des Schraubenkopfes 1.9 mm beträgt.

Mithilfe der Verwendung eines mehrgängigen Gewindes kann die Steigung s eines jeden Gewindeganges und somit der Gewindehub der Schraube vergrößert werden. Auf diese Weise kann einerseits ein bestimmter Hub mit weniger Umdrehungen beziehungsweise in kürzerer Zeit erreichbar sein, wodurch das Eindrehverhalten verbessert und das Sehnengewebe geschont wird. Andererseits steht durch die Verwendung mehrerer Gewindegänge eine hohe Oberfläche für das Anwachsen des Sehnengewebes am Sehnenanker zur Verfügung, wodurch die Fixierung der Sehne verbessert und die Rotationsstabilität erhöht wird. Das erfindungsgemäße Knochentransplantat gewährleistet somit eine gute Fixierung der Sehne und ist rasch implantierbar.

## Patentansprüche

1. Sehnenanker zur Refixation von Sehnen mit einem zylindrischen oder kegelstumpfförmigen Schraubenschaft (1) und einem Schraubenkopf (2) zur Einleitung eines Eindrehmoments, wobei sowohl der Schraubenschaft (1) als auch der Schraubenkopf (2) mit einem Außengewinde versehen sind, wobei der Sehnenanker als bohrungsfreies Knochentransplantat aus einem kortikalen Knochenmaterial gefertigt ist, wobei es sich zumindest beim Außengewinde des Schraubenschaftes um ein mehrgängiges Gewinde handelt und axiale Ausnehmungen (3) in den Außenmantel des Schraubenkopfes eingefräst sind, die in die proximale Stirnfläche des Schraubenkopfes münden, wobei die Länge des Knochentransplantats zumindest dreimal so groß wie der Durchmesser des Knochentransplantats im Fall einer zylindrischen Ausführung ist, und zumindest dreimal so groß wie der größte Durchmesser des Knochentransplantats im Fall einer kegelstumpfförmigen Ausführung des Knochentransplantats.

2. Sehnenanker nach Anspruch 1, **dadurch gekennzeichnet, dass** das mehrgängige Gewinde zwei Gewindegänge umfasst, die jeweils eine Steigung (s) zwischen 0.8 mm und 3 mm aufweisen.

3. Sehnenanker nach Anspruch 1 oder 2, **dadurch gekennzeichnet, dass** es sich bei dem Gewinde um ein Spitzgewinde handelt.

4. Sehnenanker nach einem der Ansprüche 1 bis 3, **dadurch gekennzeichnet, dass** zumindest zwei um die Schraubenkopfachse (S) verteilt angeordnete, in Richtung der Schraubenkopfachse (S) axial verlaufende und in die Stirnfläche des freien Endes des Schraubenkopfes (2) mündende Ausnehmungen (3) zur Aufnahme eines Eindrehwerkzeuges vorgesehen sind, wobei die Ausnehmungen (3) jeweils durch Seitenflächen (4) gebildet werden, die sich von einer das Außengewinde des Schraubenkopfes einhüllenden Außenmantelfläche in Richtung der Schraubenkopfachse (S) erstrecken und in einem achsnahen Flächenabschnitt ineinander übergehen.

5. Sehnenanker nach Anspruch 4, **dadurch gekennzeichnet, dass** vier symmetrisch um die Schraubenkopfachse (S) verteilt angeordnete Ausnehmungen (3) vorgesehen sind.

## Claims

1. Tendon anchor for tendon refixation, comprising a cylindrical or frustoconical screw shaft (1) and a screw head (2) for applying an insertion torque, wherein both the screw shaft (1) and the screw head (2) are provided with an external thread, wherein the tendon anchor is manufactured as a drill-free bone graft from a cortical bone material, wherein at least the external thread of the screw shaft is a multi-start thread and axial recesses (3) are milled into the outer surface of the screw head, which open into the proximal end face of the screw head, wherein the length of the bone graft is at least three times the diameter of the bone graft in the case of a cylindrical design, and at least three times the largest diameter of the bone graft in the case of a frustoconical design of the bone graft.

2. Tendon anchor according to claim 1, **characterized in that** the multi-start thread comprises two thread turns, each having a pitch (s) between 0.8 mm and 3 mm.

3. Tendon anchor according to claim 1 or 2, **characterized in that** the thread is a pointed thread.

4. Tendon anchor according to one of claims 1 to 3, **characterized in that** at least two recesses (3) for receiving a screwing-in tool are provided, which recesses (3) are distributed around the screw head axis (S), extend axially in the direction of the screw head axis (S) and open into the end face of the free end of the screw head (2), wherein the recesses (3) are each formed by lateral surfaces (4) which extend from an outer lateral surface enveloping the external thread of the screw head in the direction of the screw head axis (S) and merge into one another in a surface section close to the axis.

5. Tendon anchor according to claim 4, **characterized in that** four recesses (3) distributed symmetrically around the screw head axis (S) are provided.

## Revendications

1. Ancrage de tendon pour la refixation de tendons, avec une tige de vis (1) cylindrique ou tronconique et une tête de vis (2) destinée à appliquer un couple de vissage, dans lequel la tige de vis (1) aussi bien que la tête de vis (2) sont munies d'un filetage extérieur, l'ancrage de tendon étant fabriqué sous la forme d'un greffon osseux sans perçage fait de matière osseuse corticale, dans lequel le filetage extérieur de la tige de vis, au moins, est un filetage multiple et des évidements axiaux (3) qui débouchent dans la surface frontale proximale de la tête de vis sont fraisés dans l'enveloppe extérieure de la tête de vis, la longueur du greffon osseux étant au moins trois fois plus grande que le diamètre du greffon osseux quand la construction est cylindrique et au moins trois fois plus grande que le plus grand diamètre du greffon osseux quand la construction du greffon osseux est tronconique.

2. Ancrage de tendon selon la revendication 1, **caractérisé en ce que** le filetage multiple comprend deux spires qui présentent chacune un pas (s) compris entre 0,8 mm et 3 mm.

3. Ancrage de tendon selon la revendication 1 ou 2, **caractérisé en ce que** le filetage est un filetage triangulaire.

4. Ancrage de tendon selon l'une des revendications 1 à 3, **caractérisé en ce qu'**au moins deux évidements (3) répartis autour de l'axe de la tête de vis (S), orientés dans le sens axial dans la direction de l'axe de la tête de vis (S) et débouchant dans la face frontale de l'extrémité libre de la tête de vis (2) sont prévus pour recevoir un outil de vissage, les évidements (3) étant formés chacun par des surfaces latérales (4) qui s'étendent d'une surface d'enveloppe extérieure entourant le filetage extérieur de la tête de vis vers l'axe de la tête de vis (S) et se rejoignent dans une section de surface proche de l'axe.

5. Ancrage de tendon selon la revendication 4, **caractérisé en ce que** quatre évidements (3) répartis de façon symétrique autour de l'axe de la tête de vis (S) sont prévus.
